# EUROPEAN PATENT APPLICATION

(11) **EP 4 624 588 A1**
(43) Date of publication of application: **01.10.2025**
(21) Application number: 25162485.4
(22) Date of filing: 10.03.2025
(51) Int. Cl.: C12Q 1/6804

(54) **METHOD FOR IMMUNOLOGICAL EVALUATION OF MRNA EXPRESSION LEVEL**

(30) Priority: 19.03.2024 JP 2024042978
(71) Applicant: Ricoh Company, Ltd., Tokyo 143-8555 (JP)
(72) Inventor: MORIYAMA, Yuki, Tokyo, 143-8555 (JP); EGUCHI, Yuichi, Tokyo, 143-8555 (JP); IJICHI, Rie, Tokyo, 143-8555 (JP)
(74) Representative: Marks & Clerk LLP

(57) **Abstract**

A problem to be addressed by the present invention is to develop a method capable of immunologically simply and accurately quantitate and analyze proteins in order that the function and effect of two or more mRNAs having different sequences can be compared and evaluated in the form of the expression levels of the proteins encoded by the mRNAs. Labeled mRNAs in each of which a labeling nucleotide encoding the same labeling peptide is linked to an end of an mRNA are prepared. The respective expression levels of the labeled polypeptides expressed from the labeled mRNAs are quantitated using the same antibody against the labeling peptide.

## Description

### BACKGROUND OF THE INVENTION

### Field of the Invention

The present invention relates to a method for immunological evaluation of an mRNA expression level.

### Description of the Related Art

An mRNA vaccine is introduced into an organism through injection, and functions by the expression of a protein encoded by the mRNA. The effect of the mRNA vaccine is evaluated according to the protein expressed in an organism.

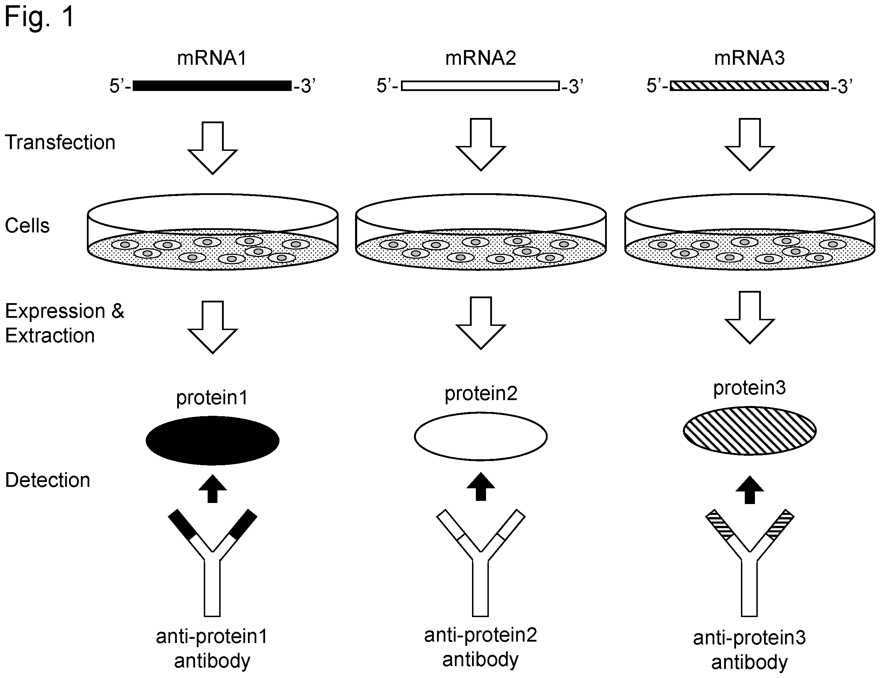

The primary function and effect of an mRNA vaccine depend on the base sequence constituting the mRNA or the length of the base sequence. For example, two different mRNA vaccines of which the respective degenerate codons encode the same protein express proteins having the same amino acid sequence. The mRNAs have different base sequences, and thus, the mRNA vaccines do not necessarily exhibit the same behavior when introduced into an organism. In a specific example, the mRNA vaccines express proteins at different levels respectively. The function and effect of an mRNA vaccine are relatively evaluated according to the expression level of a target protein expressed from an mRNA vaccine. As illustrated in FIG. 1, a protein expression level has conventionally been measured using an immunological assay with an antibody that specifically binds to a protein. Such an assay has some problems.

A first problem is that a measurement itself is impracticable without obtaining an antibody having a high specificity. For some target proteins, antibodies are commercially available, but many of such antibodies are insufficient in specificity and binding capacity, bringing about a problem in that selecting an antibody satisfactory for measurement involves a large amount of time and cost. In a case where it is difficult to obtain an antibody, for example, because the antibody is not commercially available, it is unavoidable to produce an antibody in person, bringing about a problem of entailing additional enormous time and labor.

A second problem is that, in a case where a protein as the target of measurement is present endogenously in a cell, the accuracy of detection of the protein decreases. Even if an antibody having a high specificity is used, but if a target protein derived from an mRNA introduced and an endogenous target protein are the same, the antibody is unable to distinguish both of the proteins. Accordingly, there is a problem in that it is impracticable to accurately measure the expression level of the target protein derived from the mRNA introduced.

A third problem is that the assay has a low versatility. Measuring and comparing the respective expression levels of a plurality of target proteins entails providing antibodies having a high specificity to the target proteins respectively. There is a problem in that, owing to the first problem, it is difficult to measure a protein other than the target proteins for which antibodies having a high specificity are easily available. There is also a problem in that, because the antibody-to-protein binding capacity is not necessarily the same between antibodies, it is impracticable to simply compare the respective expression levels of the target proteins.

Patent Document 1 discloses a plurality of compositions for analyzing a plurality of proteins quantitatively, the compositions including a protein-binding reagent bound to an oligonucleotide comprising a unique identifier. This protein-binding reagent can specifically bind to a protein target, thus making it possible to quantitatively analyze a plurality of protein targets in a sample. Patent Document 1 also discloses, for example, the following: a method and a kit for simultaneously and quantitatively analyzing protein targets and nucleic acid targets in a sample; and a system for preparing a labeling biomolecular reagent. This method can solve the first and the second problem. The binding capacity is not the same between each protein-binding reagent and a protein to which the reagent specifically binds, and hence, it is still impracticable to solve the third problem in that the expression levels measured are unable to be simply compared.

### SUMMARY OF THE INVENTION

The present invention is to develop and provide a highly versatile method capable of immunologically, simply and accurately making a quantitative analysis of a target protein by evaluating the functions and effects of a plurality of target mRNAs consisting of different base sequences based on each expression level of target proteins expressed from the target mRNAs.

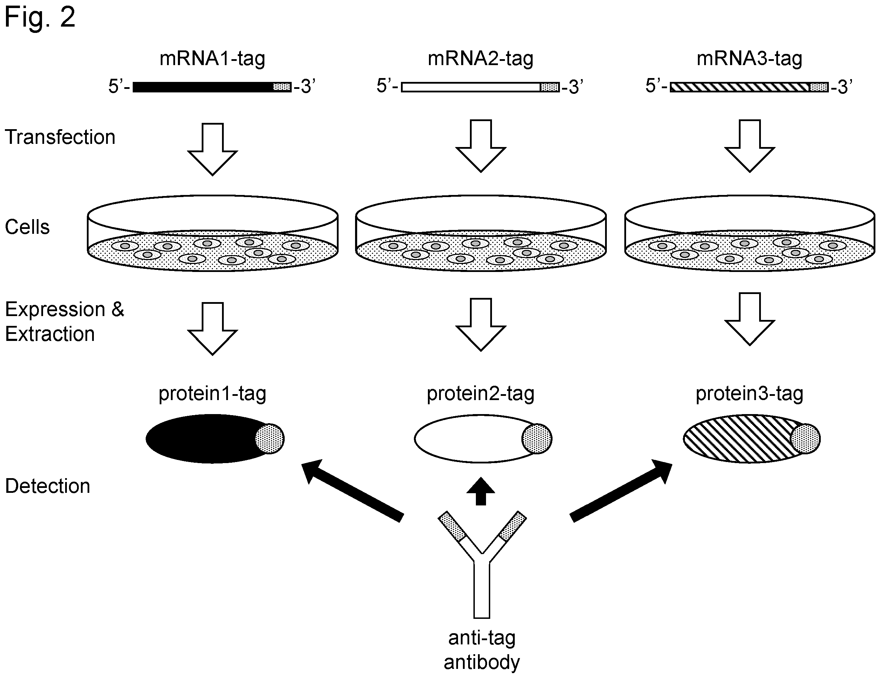

To solve the above-described problems, the present inventors have vigorously made studies, and have consequently come to develop a method using fusion mRNAs shown in FIG. 2, consisting of a plurality of target mRNAs having different base sequences and a labeling nucleotide linked to an end thereof, encoding the same labeling peptide in common. This method makes it possible that, after expressing the fusion mRNAs, all the target proteins can be quantitatively analyzed simply and accurately with one kind of common antibody that binds to the labeling peptide. Thus, the method has an extremely high versatility. The present invention is based on the result of development, and provides the following (1) and (2).
(1) A method for immunological evaluation of an mRNA expression level, comprising: an expression step of allowing labeled mRNAs to perform expression, consisting of two or more different target mRNAs; and a labeling nucleotide encoding the same labeling peptide, linked to an end of each target RNA; and a quantitation step of quantitating, using the same antibody against the labeling peptide, a labeled polypeptide expressed from each labeled mRNA in the expression step.
(2) A method for immunological evaluation of an mRNA expression level, including: an expression step of allowing a labeled mRNA to perform expression, consisting of a target mRNA and a labeling nucleotide encoding a labeling peptide, linked to an end of the target mRNA; a quantitation step of quantitating, using an antibody against the labeling peptide, a labeled polypeptide expressed from the labeled mRNA in the expression step; and an analysis step of comparing, against a reference value, the quantitative value of the labeled polypeptide obtained in the quantitation step, and analyzing the expression level of the target mRNA from the comparative result, wherein the reference value is a value obtained by quantitating, using the antibody, a labeled reference polypeptide expressed from a labeled reference mRNA comprising a reference mRNA consisting of a base sequence different from the target mRNA and the same labeling nucleotide linked to the reference mRNA.

A method for immunological evaluation according to the present invention makes it possible that the expression levels of the proteins expressed from a plurality of mRNAs consisting of different base sequences are quantitated and comparatively analyzed using one kind of antibody in common. Thus, the function and effect of each mRNA can be immunologically evaluated simply and accurately.

### BRIEF DESCRIPTION OF THE DRAWINGS

FIG. 1 is a conceptual diagram of a conventional general method for immunological measurement of an mRNA expression level;
FIG. 2 is a conceptual diagram of a method for immunologically measuring an mRNA expression level according to the present invention;
FIG. 3 is a flowchart of a method for immunological evaluation according to the present invention; and
FIG. 4 is graphs showing the results of Examples in the present invention, illustrating the measurement results of the fluorescence intensity of EGFP (FIG. 4A) and the measurement results by an immunostaining assay performed with an anti-Flag antibody (FIG. 4B), wherein both measurements were made of the expression level of EGFP in HEK293 cells after EGFP mRNA or EGFP-Flag mRNA was introduced into the cells, and wherein an HEK293 cell having no mRNA introduced thereinto was used as a control.

### DESCRIPTION OF THE EMBODIMENTS

### 1. Method (1) for immunological evaluation of mRNA expression level

### 1-1. Outline

A first aspect of the present invention is a method for immunological evaluation of an mRNA expression level. In the present invention, to evaluate each expression level of two or more target mRNAs consisting of different base sequences with the same labeling nucleotide linked to an end thereof to prepare labeled mRNAs, and the expression level of a labeled polypeptide expressed from the labeled mRNA is quantitated using the same antibody against the labeling peptides. This method makes it possible that the expression level of a target mRNA is accurately evaluated using an easily available antibody without preparing an antibody that specifically binds to each target mRNA, and without being affected by an endogenous mRNA.

### 1-2. Definitions of terms

Terms used herein are defined below.

As used herein, an "mRNA expression level" or the "expression level of an mRNA" refers to a protein-expression activity of one mRNA. This expression level is presented as the level of a protein expressed from a predetermined mRNA introduced into a cell. A protein level herein can be a relative value, such as a value of a fluorescence intensity, or can be an absolute value, such as a value of a mass.

As used herein, "immunological evaluation of an mRNA expression level" refers to using an immunological assay with an antibody to measure and evaluate the expression level of a predetermined mRNA in the form of the expression level of a protein expressed from an mRNA.

As used herein, a "target mRNA" refers to an mRNA as a target of measurement in the method according to the present invention. A polypeptide encoded by a target mRNA is herein referred to as a "target polypeptide".

As used herein, a "labeling peptide" refers to a tag peptide consisting of 7 to 40 known amino acid sequences. A labeling peptide can label a polypeptide by linking to an end of the amino acid sequence of another polypeptide. A labeling peptide herein is preferably an epitope tag.

As used herein, an "epitope tag" refers to a labeling peptide comprising at least one epitope in the amino acid sequence thereof. Specific examples of the epitope tag comprise, but are not limited to: a Flag (registered trademark) tag shown in SEQ ID NO: 1 (DYKDDDDK); an HA tag shown in SEQ ID NO: 2 (YPYDVPDYA); a 6×His tag shown in SEQ ID NO: 3 (HHHHHH); an Myc tag shown in SEQ ID NO: 4 (EQKLISEEDL); a V5 tag shown in SEQ ID NO: 5 (GKPIPNPLLGLDST); a T7 tag shown in SEQ ID NO: 6 (MASMTGGQQMG); an S tag shown in SEQ ID NO: 7 (KETAAAKFERQHMDS); an E tag shown in SEQ ID NO: 8 (GAPVPYPDPLEPR); and a Glu-Glu tag shown in SEQ ID NO: 9 (EEEEYMPME). For an epitope tag, there usually exists a known antibody (tag antibody) that specifically recognizes the epitope comprised in the epitope tag. Even if an antibody that specifically recognizes a polypeptide of a labeled polypeptide is not prepared, it is possible to immunologically detect and quantitate the labeled polypeptide, using a tag antibody against the labeling peptide.

As used herein, a "labeling nucleotide" refers to a nucleotide that encodes the labeling peptide. A labeling nucleotide is linked to an end of the base sequence of a target mRNA with the open reading frames matched to each other. An end to which a labeling nucleotide is linked can be one or both of the 5' end and the 3' end. The end is preferably the 3' end.

As used herein, a "labeled mRNA" is a target mRNA with a labeling nucleotide linked thereto, and encodes the below-described labeled polypeptide. The labeled mRNA herein can comprise a plurality of labeled mRNAs (herein often referred to as "two or more different labeled mRNAs") comprising two or more target mRNAs consisting of different base sequences with the same labeling nucleotide linked to each end thereof. Here, the "two or more target mRNAs consisting of different base sequences" comprises not only mRNAs that encode different polypeptides each other but also target mRNAs that encode the same polypeptide but have different base sequences in accordance with a difference in the codon encoding a peptide or the presence or absence of modification of a nucleobase. One labeled mRNA can comprise two or more different labeling nucleotides. In this case, the labeling nucleotides can be linked to the different ends of a target mRNA, or can be linked to the same end in series. In a case where two or more different labeled mRNAs each comprise two or more different labeling nucleotides, the kinds of the labeling nucleotides comprised in each labeled mRNA are not particularly limited as long as at least one of the labeling nucleotides is in common. The number of labeling nucleotides comprised in each labeled mRNA and the number of bases of the labeling nucleotide are desirably the same or approximately the same. The labeled mRNA can be designed in such a manner that a linker is inserted between a labeling nucleotide and a target mRNA. The linker is not particularly limited, and is preferably a linker that does not impair the steric structure or function of a target polypeptide. The linker is, for example, but not limited to, a GS linker.

As used herein, a "labeled polypeptide" refers to a polypeptide that is encoded by a labeled mRNA, and is a target of measurement in a method for immunological evaluation according to the present invention. The labeled polypeptides expressed from the two or more different labeled mRNAs respectively are herein often referred to as "two or more different labeled polypeptides". The two or more different labeled polypeptides can consist of the same amino acid sequence in some cases.

As used herein, the "reference mRNA" refers to an mRNA that consists of a base sequence different from the base sequence of a target mRNA, and serves as a comparative criterion with the target mRNA expression level of which is quantitated in a method for immunological evaluation according to the present invention. The base sequence of the reference mRNA is preferably, but not limited to, a sequence similar to the base sequence of a target mRNA, and, for example, has a base identity of 70% or more, 75% or more, 80% or more, 85% or more, or 90% or more, to the base sequence of the target mRNA. The base length of the reference mRNA is preferably, but not limited to, the same as, or different by 1 to 20 bases from, the base sequence of a target mRNA.

As used herein, a "labeled reference mRNA" is a reference mRNA with a labeling nucleotide linked thereto, and encodes the below-described labeled reference polypeptide. The labeling nucleotide linked to a labeled reference mRNA is the same labeling nucleotide linked to a labeled mRNA.

As used herein, a "labeled reference polypeptide" refers to a polypeptide encoded by a labeled reference mRNA.

As used herein, a "quantitative value" refers to the level of a labeled polypeptide expressed from a labeled mRNA. This quantitative value is a value quantitated using an antibody against a labeling peptide contained in a labeled polypeptide. This value can be a relative quantity indicated by a fluorescence intensity, luminescence intensity, turbidity, absorbance, dose of radiation, ionic strength, or concentration, or can be an absolute quantity such as the weight or volume of a labeled polypeptide included in a sample.

As used herein, a "reference value" refers to a value obtained by quantitating a labeled reference polypeptide expressed from a labeled reference mRNA. This value can be a relative value or an absolute value in the same manner as the above-described quantitative value.

### 1-3. Method

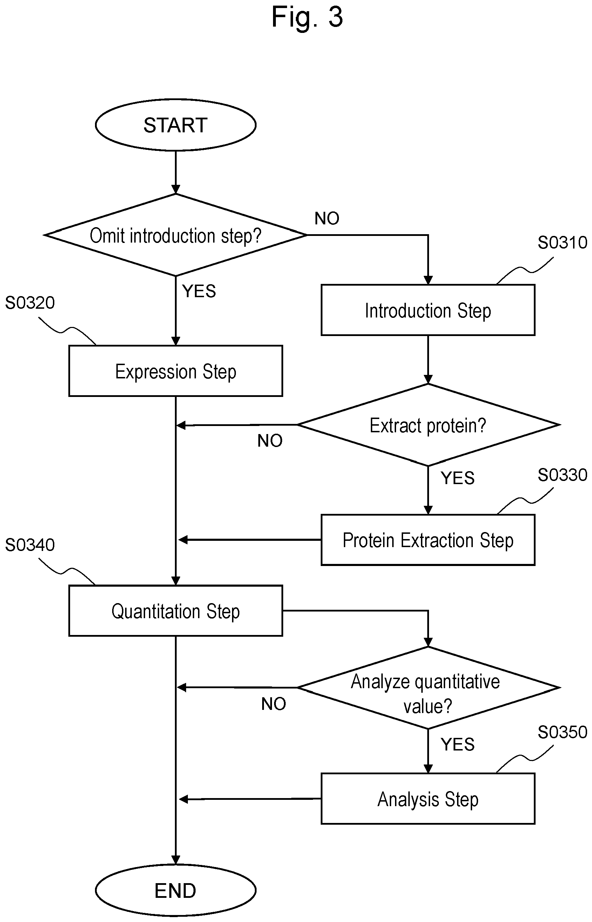

A flowchart of a method for immunological evaluation according to the present aspect is illustrated in FIG. 3. A method for immunological evaluation according to the present invention includes an expression step (S0320) and a quantitation step (S0340) as essential steps, and an introduction step (S0310), a protein extraction step (S0330), and an analysis step (S0350) as optional steps. The steps are described below.

### 1-3-1. Introduction step

The "introduction step" (S0310) is a step of introducing each of two or more different labeled mRNAs into a cell. This step is a step to be selected in a case where a labeled mRNA is to perform expression in a cell.

The preparation of a labeled mRNA to be used in this step is not particularly limited. For example, using a gene recombination technology, a labeled mRNA in which a labeling nucleotide is linked to a target mRNA can be prepared. In this case, for example, it is possible that the sequence of a target DNA corresponding to a target mRNA is inserted in an expression vector with a labeling nucleotide preliminarily contained therein, and then, is expressed in a host cell to produce a labeled mRNA of interest. It is also possible that the whole base sequence of a labeled mRNA is designed, and then, a labeled DNA consisting of the base sequence converted into a DNA is artificially synthesized *in vitro.* In this case, the resulting labeled DNA is inserted into an expression vector, and then expressed in a host cell in the same manner as described above, thus making it possible to obtain a labeled mRNA of interest. A labeled mRNA can be artificially synthesized directly *in vitro.*

A cell to be used in this step is not particularly limited. For example, bacteria, a yeast, an insect or a cultured cell thereof, a cultured animal cell, or a cultured plant cell can be utilized. Examples of the bacteria include *Escherichia coli* and *Bacillus subtilis.* Examples of the yeast include *Saccharomyces cerevisiae, Schizosaccharomyces pombe,* and *Pichia pastoris.* Examples of the insect include *Bombyx mori, Antheraea yamamai,* and *Samia cynthia.* Examples of the cultured insect cell include an Sf9 cell, Sf9 plus cell, Sf21 cell, High Five cell, and Ea4 cell. Examples of the cultured animal cell include a primary cultured cell derived from a differentiated cell such as a somatic cel, an undifferentiated stem cell, and an established cell line. Examples of the stem cell include an embryonic stem cell, mesenchymal stem cell, and induced pluripotent stem cell. Examples of the established cell line include a HeLa cell, HEK293 cell, NIH3T3 cell, CHO cell, human fibroblast, FL cell, COS-7 cell, Vero cell, L cell, and GH3 cell. Examples of the cultured plant cell include a BY-2 cell. These cells may each be any cell collected or cultured from an organism, or a genetically modified cell.

A method for introducing a labeled mRNA cell is not particularly limited. The method can be in accordance with a transformation method or a transfection method known in the art, considering the kind of a cell to be introduced and the kind of a labeled mRNA allowed to perform expression. Examples of such a method that is utilizable include an electroporation method, heatshock method, protoplast method, lipofection method, PEG (polyethylene glycol) method, calcium phosphate method, DEAE dextran method, protoplast method, particle gun method, *Agrobacterium* method, and viral infection method. For a transformation method or a transfection method, reference can be made, for example, to a gene transfer method described in Green & Sambrook, Molecular Cloning: A Laboratory Manual fourth edition, 2012, Cold Spring Harbor Laboratory Press, Cold Spring Harbor, New York.

### 1-3-2. Expression step

The "expression step" (S0320) is a step of expressing labeled polypeptides from two or more different labeled mRNAs. The present step can be performed *in vivo* in a cell having a labeled mRNA introduced thereinto, or can be performed *in vitro,* using a cell-free protein expression system. Each labeled mRNA is preferably allowed to perform expression under the same conditions. For example, in a case where the expression is performed *in vivo,* all the labeled mRNAs are allowed to perform expression, using the same kind of cell, and cultured under the same conditions.

In a case where the expression is performed *in vivo,* the present step can usually be achieved by culturing a cell having a labeled mRNA introduced thereinto. In principle, the expression of a labeled mRNA in the present step is a transient expression.

In a method for culturing a cell, cells are seeded in a medium, and then cultured under predetermined culture conditions. A medium to be used in this culture and the predetermined culture conditions can be in accordance with a culture method known in the art, depending on the kind of the cell. The culture is desirably performed under the whole germ-free conditions, in principle.

For example, in a case where *Escherichia coli* is used, *Escherichia coli* having a labeled mRNA introduced thereinto can be seeded in a known medium such as an LB medium or an ND medium, and then undergo permeation culture at 37°C.

In a case where a yeast is used, a yeast having a labeled mRNA introduced thereinto can be seeded in a known medium such as a YEP medium, YPD medium, YNB medium, or SD medium, and then undergo permeation culture at 30°C.

In a case where an insect cell is used, a known medium such as Sf900^{™}II, TC-100, or SFM-PB is optionally supplemented with serum, and then, insect cells having a labeled mRNA introduced thereinto can be seeded in the medium, and then cultured at 27°C.

In a case where a cultured animal cell such as from a mammal is used, animal cells having a labeled mRNA introduced thereinto can be seeded in a standard cell culture medium optionally supplemented with serum, and then cultured at 37°C under 5% CO₂. A "standard cell culture medium" as used herein refers to a highly versatile basic medium to be used to culture various kinds of cells mainly derived from mammals. Specific examples include an Eagle MEM (Eagle Minimum Essential Medium), DMEM (Dulbecco's Modified Eagle Medium), Ham's F10 (Ham's Nutrient Mixture F10) medium, Ham's F12 (Ham's Nutrient Mixture F12) medium, M199 medium, High Performance Medium 199, RPMI-1640 (Roswell Park Memorial Institute-1640) medium, and DMEM/F12 (Dulbecco's Modified Eagle Medium/Ham's Nutrient Mixture F12) medium. In the case of a DMEM/F 12 medium, the mixing ratio is not particularly limited. It is preferable that a DMEM and an F12 are mixed in the range of from 6:4 to 4:6 as the ratio of weight concentration of the components.

The specific composition of each of the above-described media is known in the art. A medium can be prepared on the basis of a composition described in a suitable document (for example, Kaech S. and Banker G., Nat. Protoc., 2006, 1(5): 2406-2415, in the case of a standard cell culture medium). A medium commercially available from a life science manufacturer such as Thermo Fisher Scientific Inc. or FUJIFILM Wako Pure Chemical Industries Corporation can be utilized.

The culture period differs depending on the kind of a cell to be cultured, and can be a period of time during which a labeled mRNA introduced into a cell performs expression. The culture period is usually 12 hours to 72 hours, preferably 24 hours to 48 hours.

In a case where the expression is performed *in vitro,* a labeled polypeptide is expressed from each labeled mRNA, using a cell-free protein expression system, as described above. The "cell-free protein expression system" refers to a system that expresses a protein from a gene of interest in a cell extract liquid extracted from a cell, utilizing a biomolecular translation mechanism included in the cell extract liquid. The original cell of a cell extract liquid to be used in a cell-free protein expression system can be a cell known in the art. Without limitation, for example, a rabbit reticulocyte, wheat germ, *or Escherichia coli* is suitably used. The expression of a labeled mRNA by a cell-free protein expression system can be performed in accordance with a method known in the art.

### 1-3-3. Protein extraction step

The "protein extraction step" (S0330) is an optional step of extracting a protein from a cell after the expression step in a case where the introduction step (S0310) is selected. The present step is intended to extract a labeled polypeptide expressed from a labeled mRNA in the expression step. In a case where the introduction step is selected, the present step is preferably selected together, without limitation.

**In** a method for extracting a protein from a cell, it is common that cells are lysed, and then, a protein is extracted from the cell lysate. A method for lysing a cell can be performed using an optimal known method suitably selected in accordance with the kind of a cell and the localization (in a cytoplasma, cell wall, or culture supernatant) of a labeled polypeptide of interest.

**In** a case where the cell used in the introduction step is bacteria such as *Escherichia coli,* an insect cell, or a cultured animal cell, a suitable method for lysing a cell is, for example, but not limited to, an osmotic shock method, freezing and thawing method, surfactant extraction method, or a combination of the methods. **In** the case of a cell with a cell wall, such as a yeast or a plant cell, a suitable method for lysing a cell is, for example, but not limited to, an enzyme digestion method, ultrasonic breaking method, French press method, homogenizer method, glass bead method, or a combination of the methods.

The osmotic shock method is a method in which cells are suspended in a hypotonic solution such as sterile water, and the cell membranes are broken with an osmotic pressure difference to lyse the cells. The freezing and thawing method is a method in which a cell suspension liquid is rapidly cooled with liquid nitrogen, and then thawed to break the cell membranes, whereby the cells are lysed. The surfactant extraction method facilitates the lysis of a hydrophobic protein by addition of a surfactant to thereby lyse cells, enabling the protein to be lysable, and is a most common and suitable method as a protein extraction method. A suitable surfactant to be used is a nonionic or ampholytic surfactant, without limitation. The enzyme digestion method is a method in which cells are treated with an enzyme (lysozyme, cellulase, or pectinase) specific to each cell, whereby cell walls difficult to lyse are broken, thus causing the cells to be lysed. The ultrasonic breaking method is a method in which cell membranes and cell walls are broken by ultrasonication to lyse the cells. The French press method is a method in which a cell suspension is forcibly extruded through pores under high pressure, whereby the cells are broken under a shearing force. The homogenizer method is a method in which cells, together with tissues or in a cell suspension, are mechanically homogenized to be broken. The glass bead method is a method in which cell walls or cell membranes are physically broken by collision or friction against glass beads.

Any of the above-described methods is known in the art, and in accordance with the method, a more specific method can be performed.

Without limitation, a labeled polypeptide as a target to be quantitated in the present invention is localized in a cytoplasma or a culture supernatant. Accordingly, the cell lysate prepared as described above is centrifuged, and the supernatant is collected, making it possible to obtain a protein extract liquid comprising a labeled polypeptide of interest expressed in the cell.

### 1-3-4. Quantitation step

The "quantitation step" (S0340) is a step in which a labeled polypeptide expressed from each of the two or more different labeled mRNAs in the expression step (S0320) is measured and quantitated using an antibody against a labeling peptide in common to the labeled polypeptides. The present step is an essential step that is highly characteristic and significant in a method for immunological evaluation according to the present invention. After the present step, the quantitative value of each labeled polypeptide is obtained. This quantitative value can be a relative value or an absolute value as described above, but is preferably a value obtained through measurement and quantitation based on the same reference in accordance with the same quantitation method among the labeled polypeptides.

Each labeled mRNA that encodes two or more different labeled polypeptides in the present invention are different from each other in the base sequence of the region of the target mRNA contained in the labeled mRNA, but at least one of the labeling nucleotides linked to the ends of the target mRNA is in common. Accordingly, the labeled polypeptides expressed in each labeled mRNAs in the expression step comprise the same labeling peptide. In the quantitation in the present step, an antibody that specifically recognizes and binds to this labeling peptide in common, i.e., an anti-labeling-peptide antibody is used.

As used herein, an "antibody" refers to a polypeptide that comprises a framework region (FR) and a complementarity determining region (CDR) both derived from an immunoglobulin, and has a function for specifically binding to an antigen. An "antibody" as used herein comprises not only a full-length antibody but also a functional fragment (polypeptide fragment) possessing an antigen-binding capability. The antibody can be a natural antibody or an artificial antibody.

As used herein, a "natural antibody" refers to an antibody produced in the living body of a vertebrate or an antibody consisting of the same amino acid sequence as the former antibody, and produced from an antibody-producing cell (for example, a hybridoma cell) produced artificially. Examples of the natural antibody include polyclonal antibodies and monoclonal antibodies. Without limitation, a monoclonal antibody is preferable in terms of the antibody specificity to a labeling peptide.

A "polyclonal antibody" refers to a group of a plurality of different immunoglobulins that recognize and bind to different epitopes of the same antigen. A polyclonal antibody can be obtained from a serum of an animal after immunizing the animal against the target molecule as an antigen.

A "monoclonal antibody" refers to a clonal group of single immunoglobulins. The immunoglobulins constituting a monoclonal antibody comprise a framework region in common and a complementarity determining region in common, and can recognize and bind to the same epitope of the same antigen. A monoclonal antibody can be obtained from a hybridoma derived from a single cell.

In a case where an antibody is a polyclonal antibody or a monoclonal antibody, the immunoglobulin molecule can be in any class (for example, any of IgG, IgA, IgE, IgM, IgD, and IgY). The antibody can comprise a single-chain antibody such as a VHH antibody that includes the VH region of an H-chain without an L chain as a single-chain antibody produced by a Camelidae animal does.

As used herein, an "artificial antibody" is an antibody constructed artificially. Examples include: an antibody produced by introducing a suitable mutation into the amino acid sequence of the above-described natural antibody; and besides, a single-chain antibody that does not exist in nature in principle, and has resulted from undergoing a structural modification. Specific examples of the latter artificial antibody comprise recombinant antibodies. A "recombinant antibody" refers to a chimeric antibody, humanized antibody, synthetic antibody, or antibody fragment.

A "chimeric antibody" is an antibody produced by combining the amino acid sequences of antibodies derived from different animals, and is an antibody obtained by substituting the variable region (V region) of an antibody with the V region of another antibody. For example, an antibody corresponding to a chimeric antibody is an antibody in which the variable region (V region) is derived from a mouse, and in which the C region is derived from a human, the antibody being obtained by substituting the V region of a mouse monoclonal antibody with the V region of a human antibody.

A "humanized antibody" is a graft antibody obtained by substituting the complementarity determining regions (CDR; CDR1, CDR2, and CDR3) in the variable region (V region) of an antibody of a mammal other than a human, for example, a suitable mouse with the CDRs of a human monoclonal antibody. In a chimeric antibody and a humanized antibody, the V regions of the heavy chain and the light chain are derived, or the complementarity determining regions in the V regions of the heavy chain and the light chain are derived, from an antibody of a non-human animal such as a mouse. The framework regions (FR; FR1, FR2, FR3, and FR4) in the C regions or V regions are derived, and the C regions of the heavy chain and the light chain are derived, from a human antibody. Thus, the immunoreaction with the antibody can be alleviated in the body of a human.

A "synthetic antibody" refers to an antibody synthesized using a chemical method or a recombinant DNA method. Examples comprise a monomer polypeptide molecule obtained by artificially linking one or more VLs and one or more VHs of a specific antibody via a linker peptide consisting of a suitable length and a suitable sequence, and a multimer polypeptide of the monomer polypeptide. Specific examples of such a polypeptide comprise a single-chain Fv (scFv: a single chain fragment of a variable region), diabody, triabody, and tetrabody. These synthetic antibodies are divalent to tetravalent antibody fragments with a dimeric to tetrameric structure respectively based on a single-chain Fv structure. A diabody and a more multiple body can each be a multispecific antibody. A "multispecific antibody" refers to a multivalent antibody, i.e., an antibody having a plurality of antigen-binding sites in one molecule, in which antibody each antigen-binding site bind to different epitopes.

Examples of the "antibody fragment" comprise Fab, F (ab')₂, and Fv.

An antibody to be used in the present step can be a modified antibody (modified labeled antibody). **In** particular, in the below-described case where an anti-labeling-peptide antibody is recognized as a primary antibody, and a secondary antibody that specifically binds to the primary antibody is used in the quantitation of a labeled polypeptide in the present step, the secondary antibody is preferably a modified antibody.

The modification of an antibody comprises a functional modification or a labeling modification. Examples of the functional modification comprise glycosylation, acetylation, formylation, amidation, phosphorylation, and PEGylation. Examples of the labeling modification comprise labeling with a fluorescent dye (fluorescein, FITC, rhodamine, Texas red, Cy3, or Cy5), fluorescent protein (for example, PE, APC, or GFP), enzyme (for example, horseradish peroxidase, alkaline phosphatase, or glucose oxidase), radioisotope (for example, ³H, ¹⁴C, or ³⁵S), biotin, or (strept)avidin.

In the present step, a labeled polypeptide expressed is quantitated using an immunological assay, from a cell-free protein expression system solution obtained after the expression step or from a protein extract liquid obtained after the protein extraction step.

The "immunological assay" is a method as follows: a target molecule is an antigen; an antibody that specifically binds to the target molecule is used to form an immune complex with the target molecule; and the target molecule is detected and quantitated. In a method for immunological evaluation according to the present invention, a labeling peptide comprised in a labeled polypeptide corresponds to the target molecule, as described above. Accordingly, a labeled polypeptide is detected and quantitated using an anti-labeling-peptide antibody.

Examples of the immunological assay comprise an enzyme immunoassay, fluoroimmunoassay, luminescent immunoassay, surface plasmon resonance assay (SPR method), quartz crystal microbalance (QCM) assay, radioimmunoassay (RIA), immunonephelometric assay, latex agglutination immunoassay, latex nephelometric assay, particle agglutination assay, gold colloid assay, capillary electrophoresis assay, Western blotting assay, and immunohistochemical analysis (immunostaining) assay. Any of these assays is a known assay, and in principle, can be performed in accordance with a usual method in the art. For example, reference can be made to a method described in the following: Current protocols in Protein Sciences, 1995, John Wiley & Sons Inc.; Current protocols in Immunology, 2001, John Wiley & Sons Inc.; Green & Sambrook, Molecular Cloning, fourth edition, 2012, Cold Spring Harbor Laboratory Press, Cold Spring Harbor, New York; Japan Society of Clinical Pathology, "Immunoassay for Clinical Test-Technology and Application", The Japanese Journal of Clinical Pathology, Special Issue, No. 53, 1983, The Clinical Pathology Press; Eiji Ishikawa et al., Eds., Enzyme Immunoassay, third edition, 1987, Igaku-Shoin; Tsunehiro Kitagawa et al., Eds., "Enzyme Immunoassay", Proteins, Nucleic Acids, and Enzymes, Supp. No. 31, 1987, Kyoritsu Shuppan; Irie Minoru Ed., Radioimmunoassay, 1974, Kodansha Scientific; Irie Minoru Ed., A Sequel to Radioimmunoassay, 1979, Kodansha Scientific; Kazuhiro Nagata and Hiroshi Handa, Eds., Real-Time Analytical and Experimental Method of Biomolecular Interactions, 1988, Springer-Veriag, Tokyo; and Toyosaka Moriizumi and Takamichi Nakamoto, Sensor Engineering, 1997, Shokodo.

The "enzyme immunoassay" is a method in which a primary antibody bound to a target molecule is detected via a modified labeled secondary antibody, and the target molecule is quantitated according to a color optical density and a fluorescence intensity generated from the modified labeling. Examples of the assay comprise a method in which an anti-labeling-peptide antibody as a primary antibody bound to a labeling peptide is captured by a labeled secondary antibody that binds to the primary antibody, and the labeled polypeptide comprising the labeling peptide is indirectly measured on the basis of a signal intensity from the label. An ELISA and a sandwich ELISA fall under this method.

The "surface plasmon resonance (SPR) assay" is a method in which an adsorbate on the surface of a thin metal film is detected and quantitated with a high sensitivity, utilizing the phenomenon of surface plasmon resonance in which varying the incident angle of a laserbeam radiated to the thin metal film causes the intensity of reflected light to be markedly attenuated at a specific incident angle (resonance angle). In the present invention, for example, an anti-labeling-peptide antibody is solid-phased on the surface of a thin metal film; the other portions of the surface of the thin metal film are blocked; then, a cell-free protein expression system solution or a protein extract liquid is run on the surface of the thin metal film; and from a difference between the values measured before and after the sample is run, the labeled polypeptide can be detected and quantitated. The detection and quantitation by the surface plasmon resonance assay can be performed, for example, utilizing an SPR sensor commercially available from Biacore AB.

The "quartz crystal microbalance (QCM) assay" is a mass measurement method as follows: when a substance is adsorbed on the surface of an electrode attached to a quartz crystal resonator, the resonance frequency of the quartz crystal resonator decreases in accordance with the mass of the substance; and this phenomenon is utilized to quantitatively grasp a trace amount of the adsorbate in accordance with the level of variation in the resonance frequency. In the detection and quantitation in this method, a target molecule can be detected and quantitated in the same manner as in the SPR assay, utilizing a commercially available QCM sensor. In the present invention, for example, a labeled polypeptide can be quantitated in accordance with an antigen-antibody reaction between an anti-labeling-peptide antibody solid-phased on the surface of the electrode and a labeling peptide comprised in the labeled polypeptide in a sample.

An anti-labeling-peptide antibody to be used in the present step is determined in accordance with a labeling peptide comprised in a labeled polypeptide. For example, in a case where the labeling peptide is a Flag tag, an anti-labeling-peptide antibody to be used can be an anti-Flag antibody. Generally, in a case where the labeling peptide is an epitope tag, specific antibodies against various epitope tags are commercially available from various life science manufacturers, and can be utilized.

Two or more different labeled polypeptides can each comprise two or more labeling peptides that are partly the same. In the case of three or more different labeled polypeptides, each labeled polypeptide can have different combinations of labeling peptides, enabling the labeled polypeptides to be quantitated in different combinations. For example, in a case where, after three or more different labeled mRNAs perform expression, all the labeled polypeptides expressed can be quantitated on the basis of the same reference, using an antibody against a labeling peptide in common to all the labeled polypeptides. After three or more different labeled mRNAs perform expression, specific two labeled polypeptides out of the labeled polypeptides expressed on the basis of the same reference can be quantitated using an antibody against a labeling peptide in common to the two labeled polypeptides.

### 1-3-5. Analysis step

The "analysis step" (S0350) is a step in which each quantitative value of the two or more different labeled polypeptides, obtained in the quantitation step (S0340), are compared to analyze the expression level of a target mRNA contained in each of the two or more different labeled mRNAs that encode each labeled polypeptide.

Comparison of the quantitative values obtained from the two or more different labeled polypeptides is not particularly limited. As described above, these quantitative values are values measured and quantitated on the basis of the same reference, using the same quantitation method among the labeled polypeptides, and thus can be compared directly. Accordingly, comparing the quantitative values of the labeled polypeptides makes it possible to determine large and small of each value.

The large and small of the quantitative value of a labeled polypeptide reflects the expression level of a target mRNA contained in a labeled mRNA encoding the labeled polypeptide. Accordingly, the comparison results of the quantitative values among two or more different labeled polypeptides are used to analyze the large and small of the expression levels of target mRNAs contained in each labeled mRNA, thus making it possible to determine which target mRNA affords a high expression level. This makes it possible that, for example, in screening of an mRNA vaccine, a target mRNA vaccine capable of achieving a highest expression level in a cell can be selected from a plurality of target mRNA vaccines consisting of different base sequences encoding the same amino acid sequence.

### 2. Method (2) for immunological evaluation of mRNA expression level

### 2-1. Outline

A second aspect of the present invention is a method for immunological evaluation of an mRNA expression level in the same manner as the first aspect. In the present aspect, the expression levels of two or more different target mRNAs are not comparatively evaluated, but the expression level of a target mRNA is evaluated by comparison with a reference value. This method makes it possible that, even with one kind of target mRNA, the expression level of the target mRNA is accurately evaluated, in the same manner as in the first aspect, using an easily available antibody without preparing an antibody that specifically binds to each target mRNA, and without being affected by an endogenous mRNA.

2-2. Method

A flowchart of a method for immunological evaluation according to the present aspect is illustrated in FIG. 3 in the same manner as in the first aspect. That is, a method for immunological evaluation according to the present aspect also comprises an expression step (S0320) and a quantitation step (S0340) as essential steps, and an introduction step (S0310), a protein extraction step (S0330), and an analysis step (S0350) as optional steps. These steps are basically in accordance with the details described about the corresponding steps in the first aspect. Accordingly, the same details as in the first aspect are omitted. Here, the methods and structures characteristic of the present aspect are specifically described below.

### 2-2-1. Introduction step

The "introduction step" (S0310) is a step of introducing, into a cell, a labeled mRNA in which a labeling nucleotide encoding a labeling peptide is linked to an end of a target mRNA. The present step can be basically the same as the introduction step (S0310) in the first aspect except that the labeled mRNA as a subject of introduction can be of one kind.

### 2-2-2. Expression step

The "expression step" (S0320) is a step of allowing a labeled mRNA to perform expression. The present step can also be basically the same as the expression step (S0320) in the first aspect except that the labeled mRNA as a subject of expression can be of one kind.

### 2-2-3. Protein extraction step

The "protein extraction step" (S0330) is an optional step of extracting a protein from a cell after the expression step in a case where the introduction step (S0310) is selected. The present step can be the same as the protein extraction step (S0330) in the first aspect.

### 2-2-4. Quantitation step

The "quantitation step" (S0340) is a step of quantitating, using an antibody against the labeling peptide, a labeled polypeptide expressed from the labeled mRNA in the expression step (S0320). The present step can also be basically the same as the quantitation step (S0340) in the first aspect except that the labeled mRNA as a subject of expression can be of one kind.

### 2-2-5. Analysis step

The "analysis step" (S0350) is a step of comparing, against a reference value, the quantitative value of the labeled polypeptide obtained in the quantitation step, and analyzing the expression level of a target mRNA from the result of comparing the values. The present step is a characteristic step most different from a method for immunological evaluation in the first aspect. In the first aspect, the quantitative values of two or more different labeled polypeptides are compared and analyzed each other. In the present step, the subject of comparison with a quantitative value is a reference value. The present step is very different from the first aspect in that the expression level of a target mRNA is analyzed on the basis of the result of comparison with a reference value.

The reference value is a value obtained by quantitating a labeled reference polypeptide. The labeled reference polypeptide is a polypeptide expressed from a labeled reference mRNA in which a labeling nucleotide is linked to a reference mRNA. If neither the reference mRNA nor the labeling nucleotide is limited, the labeled reference polypeptide comprises enormous kinds thereof. Because of this, a great number of reference values can exist. These reference values, preliminarily obtained and stored as a database, can be optionally used when the present aspect is performed.

A reference value to be used in the present step is a value obtained by quantitating a labeled reference polypeptide, using "the same antibody as an antibody used to quantitate a labeled polypeptide" in the quantitation step (S0340). That is, in the present step, a reference value is preferably selected on the basis of a labeled mRNA encoding a labeled polypeptide to be quantitated in the quantitation step. For example, the reference mRNA has a base sequence different from the base sequence of a target mRNA. The labeling nucleotide encodes the same labeling peptide as the labeled polypeptide. The labeling nucleotide is measured and quantitated on the basis of the same reference as the labeled polypeptide is. A reference value that satisfies these conditions can be, for example, selected and obtained from the above-described database.

Two or more reference values can be used in the present step, target to satisfying the conditions.

Comparison between the quantitative value and the reference value can be in accordance with the analysis step (S0350) in the first aspect. As described above, the reference value is a value measured and quantitated on the basis of the same reference, using the same method as the quantitative value. Thus, the direct comparison makes it possible to determine the magnitude of the quantitative value with respect to the reference value.

In the same manner as in the first aspect, the magnitude of the quantitative value of a labeled polypeptide reflects the expression level of a target mRNA comprised in a labeled mRNA encoding the labeled polypeptide. From the result of comparison with the reference value, the magnitude of the expression level of a target mRNA comprised in a labeled mRNA can be analyzed.

In the present step, a reference value can be used as a "cutoff value", for example, for classification of quantitative values. For example, in a case where the reference value is a value based on a reference mRNA that exhibits a lowest desirable expression level to function as an mRNA vaccine, and in a case where the reference value is lower than this quantitative value, the target mRNA can be determined to be an unsuitable mRNA vaccine. For example, in a case where the overexpression of an mRNA vaccine can cause a side effect to a human body, an upper-limit reference value exhibiting the expression level and a lower-limit reference value exhibiting a functional lowest desirable expression level are used to verify whether the quantitative value is in the range of from the lower-limit reference value to the upper-limit reference value. Thus, the effect and safety of the mRNA vaccine can be determined.

In the present step, the reference value can be used to determine a statistically significant difference from a quantitative value. Specifically, for example, the function of a target mRNA can be evaluated and determined according to whether the quantitative value is statistically significantly higher than the reference value.

As used herein, the phrase "statistically significant" means that the critical rate (level of significance) of a value obtained is small, specifically p < 0.05 (less than 5%), p < 0.01 (less than 1%), or p < 0.001 (less than 0.1%). Here, the "p(-value)" represents a probability at which a hypothesis is accidentally true in a hypothesized distribution of statistics in a statistical test. Accordingly, a smaller p-value means that the hypothesis is closer to truth. The phrase "statistically significant difference" means that, when a difference between a measured value of a test target and a measured value of a population is statistically processed, the difference between both is significant. As a testing method for statistical processing, a known test method capable of judging any significance can be suitably used, and the method is not particularly limited. For example, the Student t-test method can be used.

### Examples

### (Purpose)

To demonstrate that a method according to the present invention is a method capable of grasping the expression level of an actual protein, the same proteins EGFPs are compared and examined by measuring the fluorescence intensity and by using an immunostaining assay with an antibody against a labeling peptide.

### (Method)

### (1) Preparation of mRNAs

Two kinds of mRNAs used in Examples were as follows: an EGFP-GS-Flag mRNA synthesized using an unmodified nucleic acid; and an mEGFP-GS-Flag mRNA synthesized in a state in which all the Us (uracils) in an EGFP-GS-Flag mRNA were replaced with modified nucleic acids N1-methyl-psudo-U. Any of the mRNAs had the base sequence of SEQ ID NO: 11 in such a manner that a Flag tag was linked to the 3' end of the EGFP of SEQ ID NO: 10 via a GS linker of SEQ ID NO: 12. SEQ ID NOs: 10 to 12 can be RNAs in some cases, but are described as DNA sequences in the sequence listing.

A DNA template with a PolyA tail was produced with a Tailed PCR, using, as a template, a plasmid including the base sequence of EGFP-GS-Flag mRNA of SEQ ID NO: 11. The DNA template was agarose-electrophoresed. Out of the template, a band of size of interest was cut out and purified using a Gel Extraction kit (Qiagen N.V.). Using an unmodified nucleic acid and a modified nucleic acid, an mRNA was synthesized through *in vitro* transcription. For the *in vitro* transcription, T7 RNA Polymerase Mix (New England Biolabs, Inc.) was used. The mRNA was treated with DNase, and then, purified with a Monarch RNA Cleanup Kit (New England Biolabs, Inc.). The mRNA underwent a capping reaction using a Vaccinia Capping Enzyme (New England Biolabs, Inc.) and an mRNA Cap 2'-O-Methyltransferase (New England Biolabs, Inc.), and then, purified using a Monarch RNA Cleanup Kit (New England Biolabs, Inc.) again. The concentration of the mRNA was measured, and then, the mRNA was used for introduction.

### (2) Introduction of mRNAs

HEK293 cells were seeded in a 96-well plate in such a manner that 20,000 cells were comprised in each well. The cells were cultured at 37°C under 5% CO₂ overnight. A medium used was a DMEM (Thermo Fisher Scientific Inc.) comprising 10% FBS (Thermo Fisher Scientific Inc.).

A mixture was prepared in such a manner that 100 ng of each mRNA prepared in (1) and 0.2 µL of Lipofectamine Messenger MAX Reagent (Thermo Fisher Scientific Inc.) were comprised in 10 µL of Opti-MEM (Thermo Fisher Scientific Inc.) per well. The resulting mixture was added to each cell of the 96-well plate to be introduced into the HEK293 cells. Here, the resulting mixture was introduced into three wells per kind of mRNA to serve at N = 3. A negative control into which no mRNA was introduced was provided in three wells in the same manner. Then, a culture was performed in each dish at 37°C under 5% CO₂ for 24 hours, and the mRNAs introduced into each cell were allowed to perform expression.

### (3) Fluorometry

With part of the three samples, the fluorescence intensity of the EGFP in the cells was measured using a cell counter NC3000 (M&S TechnoSystems, Inc.).

### (4) Immunostaining

With part of the three samples, the expression level of the miRNAs introduced was measured using an immunostaining assay with an anti-Flag antibody. Each sample was fixed with 4% PFA, and blocked with 3% BSA, and then, used for detection with a 1/1000-fold diluted Flag antibody (Sigma-Aldrich Co. LLC). As a secondary antibody, a 1/2000-fold diluted fluorescence-labeled antimouse IgG (H+L) antibody (Thermo Fisher Scientific Inc.) was used.

### (Results)

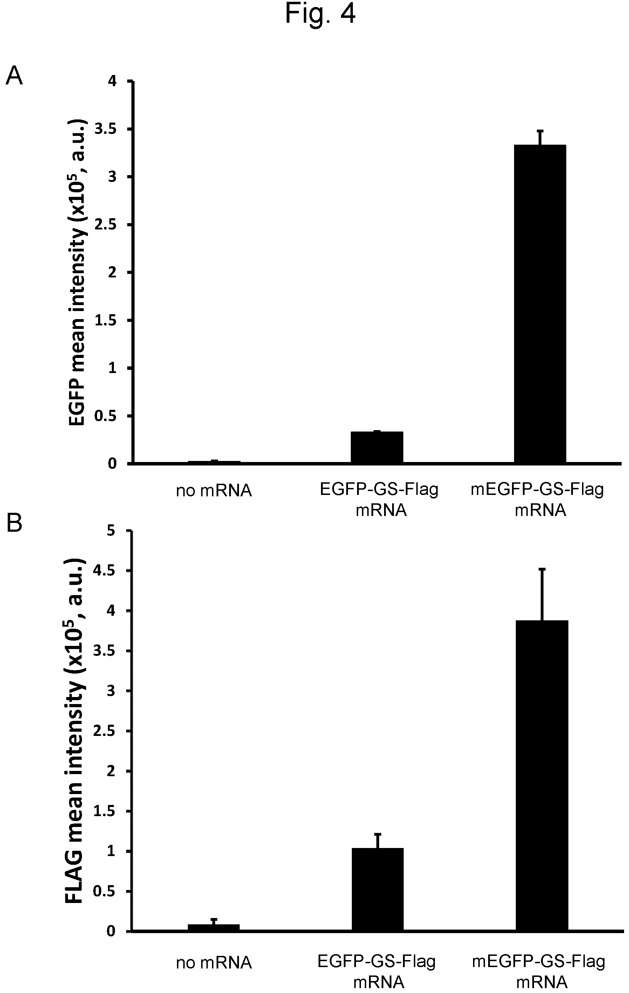

The results are illustrated in FIG. 4. FIG. 4A graphs the measurement results of the fluorescence intensity of the EGFP in each sample, and FIG. 4B graphs the immunostaining assay result obtained using an anti-Flag antibody.

FIG. 4A is as follows: the fluorescence of the intensity of the EGFP in each sample was measured; and as a result, in a case where an EGFP-GS-Flag mRNA synthesized using a transfection method with an unlabeled nucleic acid was introduced into the HEK293 cells (the lane: EGFP-GS-Flag mRNA), the expression of the EGFP protein was verified, compared with the mock (lane: no mRNA) in which no mRNA was introduced. In the HEK293 cells into which the mEGRP-GS-Flag mRNA synthesized using a modified nucleic acid was introduced (the lane: mEGFP-GS-Flag mRNA), a noticeably high level of EGFP expression was verified, compared with the EGFP-GS-Flag mRNA synthesized using an unlabeled nucleic acid.

FIG. 4B is as follows: a fluorescence intensity was measured using an immunostaining assay with an anti-Flag antibody used for the same sample; and as a result, the same results as in FIG. 4A were obtained.

The above-described results have revealed that a measurement made of a labeled polypeptide using a method for immunological evaluation according to the present invention reflects the expression level of the labeled polypeptide in a cell. Accordingly, the results have demonstrated that using a method for immunological evaluation of an mRNA expression level according to the present invention makes it possible to accurately quantitate and compare the expression levels of each labeled polypeptide, using one kind of antibody that specifically recognizes the labeling peptide in common to two or more different labeled polypeptides.

### Citation List

### Patent Document

Patent Document 1: Japanese Translation of PCT International Application Publication No. JP-T-2020-502255

Sequence Listing

## Claims

1. A method for immunological evaluation of an mRNA expression level, comprising:
an expression step of allowing labeled mRNAs to perform expression, consisting of two or more different target mRNAs and a labeling nucleotide encoding the same labeling peptide, linked to an end of each target RNA; and
a quantitation step of quantitating, using the same antibody against the labeling peptide, a labeled polypeptide expressed from each labeled mRNA in the expression step.

2. The method according to claim 1, further comprising
an analysis step of comparing the quantitative values of each labeled polypeptide obtained in the quantitation step, and analyzing the expression level of each target mRNA from the comparative results.

3. The method according to claim 1 or 2, wherein the labeled mRNAs are artificially synthesized *in vitro.*

4. The method according to claim 3, wherein the labeling peptide is an epitope tag.

5. A method for immunological evaluation of an mRNA expression level, comprising:
an expression step of allowing a labeled mRNA to perform expression, consisting of a target mRNA and a labeling nucleotide encoding a labeling peptide, linked to an end of the target mRNA;
a quantitation step of quantitating, using an antibody against the labeling peptide, a labeled polypeptide expressed from the labeled mRNA in the expression step; and
an analysis step of comparing, against a reference value, the quantitative value of the labeled polypeptide obtained in the quantitation step, and analyzing the expression level of the target mRNA from the comparative result,
wherein the reference value is a value obtained by quantitating, using the antibody, a labeled reference polypeptide expressed from a labeled reference mRNA comprising a reference mRNA consisting of a base sequence different from the target mRNA and the same labeling nucleotide linked to the reference mRNA.

6. The method according to claim 5, wherein the labeled mRNA and the labeled reference mRNA are artificially synthesized *in vitro.*

7. The method according to claim 6, wherein the labeling peptide is an epitope tag.
